# EUROPÄISCHE PATENTSCHRIFT

(11) **EP 2 536 442 B1**
(45) Veröffentlichungstag und Bekanntmachung des Hinweises auf die Patenterteilung: **27.07.2016**
(21) Anmeldenummer: 11702002.4
(22) Anmeldetag: 27.01.2011
(51) Int. Cl.: A61L 2/26, B67C 7/00, A61C 19/00

(54) **AUFNAHMEBEHÄLTER FÜR ÄRZTLICHE, INSBESONDERE ZAHNÄRZTLICHE INSTRUMENTE UND ZUGEHÖRIGES PFLEGESYSTEM**
RECEPTACLE FOR MEDICAL, ESPECIALLY DENTAL INSTRUMENTS AND ASSOCIATED MAINTENANCE SYSTEM
RÉCEPTACLE POUR INSTRUMENTS MÉDICAUX, NOTAMMENT DENTAIRES, ET SYSTÈME D'ENTRETIEN ASSOCIÉ

(30) Priorität: 17.02.2010 DE 102010002024
(43) Veröffentlichungstag der Anmeldung: 26.12.2012
(73) Patentinhaber: Kaltenbach & Voigt GmbH, 88400 Biberach (DE)
(72) Erfinder: HECKENBERGER, Hans, 88433 Aßmannshardt (DE); WIEK, Hans-Dieter, 88454 Hochdorf 2 (DE); GUGEL, Bernd, Ulm 89079 (DE)
(74) Vertreter: Thun, Clemens
(86) Internationale Anmeldenummer: PCT/EP2011/051119
(87) Internationale Veröffentlichungsnummer: WO 2011/101213

(56) Entgegenhaltungen:
- WO-A2-2010/115182
- US-A- 4 327 060
- US-A1- 2006 196 728

## Beschreibung

Die vorliegende Erfindung betrifft einen Aufnahmebehälter für ärztliche, insbesondere zahnärztliche Instrumente, wobei der Behälter zur Verwendung in einem Wiederaufbereitungsgerät vorgesehen ist, welches eine Kammer zur Aufnahme des Behälters sowie Mittel zum Desinfizieren, Sterilisieren und/oder Pflegen der Instrumente aufweist.

Bei ärztlichen oder zahnärztlichen Handstücken handelt es sich um rohrförmige Teile, die der Arzt während der Behandlung als Griffhülse ergreift. Ein üblicherweise in der zahnmedizinischen Praxis verwendetes Handstück ist ein sogenanntes Bohrhandstück, das an seinem vorderen Ende ein Behandlungswerkzeug, insbesondere einen Bohrer trägt und mit seinem hinteren Ende mittels einer Kupplung mit einem Versorgungsschlauch gekoppelt ist. Durch das Handstück erstrecken sich Versorgungsleitungen für Energie zum Antrieb des Behandlungsinstruments sowie Fluidleitungen für Behandlungsmedien, beispielsweise Luft und/oder Wasser. Unterschieden wird oftmals zwischen sogenannten Turbinen-Handstücken, bei denen zur Versorgung einer im vorderen Endbereich angeordneten Turbine Druckluft vorgesehen ist, und sogenannten Motor-Handstücken, welche als Antriebseinheit einen Elektromotor aufweisen.

Zur Aufrechterhaltung der Funktion der Handstücke bedarf es von Zeit zu Zeit einer Pflege, insbesondere der drehbar gelagerten Antriebselemente. Ferner führen die in der zahnärztlichen Praxis immer weiter ansteigenden Hygieneanforderungen dazu, dass eine Aufbereitung von Handstücken in regelmäßigen zeitlichen Abständen zu erfolgen hat. Die erfolgreiche Aufbereitung und Einhaltung der entsprechenden Vorgaben muss hierbei durch den Zahnarzt lückenlos dokumentiert werden, was einen nicht unerheblichen personellen und organisatorischen Aufwand nach sich zieht.

Eine manuelle Wiederaufbereitung zahnärztlicher Handstücke erfolgte bislang dadurch, dass die Instrumente nach Gebrauch am Patienten zunächst sprühdesinfiziert und äußerlich abgewaschen wurden. Eine Innenreinigung der Instrumente wurde hingegen in der Regel nicht durchgeführt. Zwischenzeitlich existieren auf dem Markt allerdings Reinigungs- und Desinfektions-Geräte, in denen die Instrumente aufbereitet werden, bevor sie einer Ölpflege unterzogen werden. Die maschinelle Aufbereitung bringt deutliche Vorteile gegenüber einem manuellen Pflegen der Instrumente mit sich, da nur ein maschinelles Verfahren eine sichere und reproduzierbare Reinigung und Pflege ermöglicht.

Die bislang bekannten Geräte können allerdings in der Regel lediglich für einzelne Aufbereitungsschritte benutzt werden, sodass jeweils separat eine Reinigung, eine Pflege und eine Sterilisation durchgeführt werden muss. Die Gesamtheit der hierfür erforderlichen Geräte nimmt einen relativ großen Platz in Anspruch, wobei für jedes der Geräte jeweils elektrische, pneumatische und fluidische Anschlüsse erforderlich sind. Die Realisierung einer vollständigen maschinellen Aufbereitung zahnärztlicher Instrumente mittels einzelner Geräte ist dementsprechend sehr umständlich und mit einem hohen Kostenaufwand verbunden.

Ein weiterer Nachteil besteht darin, dass die einzelnen Geräte in der Regel nicht untereinander vernetzt sind, weshalb ein Datenaustausch zwischen den Geräten nicht erfolgen kann. Dies führt wiederum zu einem Mehraufwand des Bedienpersonals, da keine durchgängig automatische Dokumentation der Instrumentenaufbereitung erstellbar ist. Ferner müssen in Zwischenschritten die Instrumente von Gerät zu Gerät manuell weiterbefördert werden, was mit einem intensiven Personaleinsatz und einem großen Zeitbedarf verbunden ist.

Es sind dementsprechend zwischenzeitlich Geräte bekannt, bei denen ein entsprechendes Wiederaufbereiten bzw. Pflegen der zahnärztlichen Instrumente automatisch erfolgt. Beispielsweise sind Geräte auf dem Markt, bei denen die Instrumente auf Kupplungen aufgesetzt werden, welche in eine Spülkammer ragen. Über die Kupplungen können Reinigungs- und Pflegemedien in das Innere der Instrumente eingebracht werden. Die Außenseite der Instrumente wiederum wird durch Medien gereinigt, welche durch in Spülkammer befindliche Düsen oder dergleichen eingebracht werden.

Um derartige Pflegegeräte mit den wiederaufzubereitenden Instrumenten zu bestücken, müssen diese auf die entsprechenden Kupplungen aufgesetzt werden. Dabei muss darauf geachtet werden, dass insbesondere in dem Zustand, in dem die Instrumente noch nicht gereinigt sind, keine Verletzung des Personals durch die Geräte erfolgt, da andernfalls eine verhältnismäßig hohe Infektionsgefahr besteht. Dementsprechend ist dieser Vorgang mit hoher Genauigkeit durchzuführen und nimmt eine entsprechende Zeit in Anspruch.

Eine Lösung dieses Problems ist aus der EP 0 638 298 A1 bekannt, in der eine Kassette zur Aufnahme zahnärztlicher Instrumente beschrieben wird. Diese Kassette weist entsprechende Kupplungen auf, auf welche die Instrumente aufgesetzt werden. Die gesamte Kassette wird dann in ein entsprechendes Wiederaufbereitungsgerät eingesetzt. Am Umfang der Kassette sind ferner Durchgangsbohrungen ausgebildet, durch welche entsprechende Sprühdüsen des Wiederaufbereitungsgeräts ragen, um die Instrumente zu reinigen. Auch hier ist allerdings der Aufwand zum Einsetzen der Kassette in das Gerät verhältnismäßig hoch. Ferner werden die Öffnungen ausschließlich durch eine Klappe überdeckt, sodass der Innenraum der Kassette nur unzureichend vor äußeren Einflüssen geschützt ist.

Aus der US 2006/0196728 A1 ist ein Aufbereitungsgerät für medizinische Handstücke bekannt, bei dem die Handstücke an Adaptern angeschlossen werden, die sich an der Unterseite eines Behälterdeckels befinden. Zum Reinigen wird dann der Behälter mit dem Behälterdeckel abgedeckt und in das Gerät eingesetzt. Die Zufuhr von Reinigungsmitteln erfolgt über Kanäle, die den Behälterdeckel durchsetzen.

Aus der US 4,327,060 ist ein Sterilisationsbehälter bekannt, der für eine chemische Sterilisation geschlossen werden kann und für eine Dampfsterilisation geöffnet werden kann.

Der vorliegenden Erfindung liegt dementsprechend die Aufgabe zugrunde, eine neuartige Möglichkeit zum Anordnen insbesondere zahnärztlicher Instrumente in einem Wiederaufbereitungsgerät anzugeben, wobei einerseits das Anordnen der Instrumente erleichtert werden und andererseits ein möglichst ein guter Schutz der bereits gereinigten Instrumente vorliegen soll.

Die Aufgabe wird durch einen Aufnahmebehälter, der die Merkmale des Anspruchs 1 aufweist, gelöst. Vorteilhafte Weiterbildungen der Erfindung sind Gegenstand der abhängigen Ansprüche.

Der erfindungsgemäßen Lösung liegt die Idee zugrunde, einen Aufnahmebehälter zu schaffen, der einerseits geöffnet und andererseits geschlossen werden kann. Im geöffneten Zustand ist dabei der Behälter derart ausgestaltet, dass darin angeordnete Instrumente für die entsprechenden Reinigungsmittel eines Wiederaufbereitungsgeräts zugänglich sind. Im geschlossenen Zustand hingegen soll der Innenraum des Behälters möglichst hermetisch gegenüber der Umgebung abgeschlossen sein. Das Öffnen und Schließen des Behälters kann dabei durch das Wiederaufbereitungsgerät selbst erfolgen, wozu dieses besondere Mittel aufweist, um den in die Kammer eingesetzten Behälter entsprechend zu betätigen.

Dementsprechend wird erfindungsgemäß ein Aufnahmebehälter für ärztliche, insbesondere zahnärztliche Instrumente vorgeschlagen, wobei der Behälter zur Verwendung in einem Wiederaufbereitungsgerät vorgesehen ist, welches eine Kammer zur Aufnahme des Behälters sowie Mittel zum Desinfizieren, Sterilisieren und/oder Pflegen der Instrumente aufweist. Der Behälter ist dadurch gekennzeichnet, dass er von einem Transportzustand, in dem er geschlossen ist, in einen Pflegezustand, in dem er derart geöffnet ist, dass darin angeordnete Instrumente zugänglich sind, überführbar ist, wobei der Behälter derart mit dem Wiederaufbereitungsgerät koppelbar ist, dass dieses in der Lage ist, den in die Kammer eingesetzten Behälter wahlweise von dem Transportzustand in den Pflegezustand bzw. umgekehrt zu überführen.

Der mit den Instrumenten bestückte Behälter kann also dann als Einheit in das Wiederaufbereitungsgerät eingesetzt werden, sodass dieser Vorgang sehr schnell und einfach durchzuführen ist. Gleichzeitig besteht die Möglichkeit, bereits während des Betriebs des Wiederaufbereitungsgeräts einen weiteren Transportbehälter entsprechend zu bestücken, sodass anschließend lediglich ein kurzer Austausch der Behälter erforderlich ist. Das Wiederaufbereitungsgerät kann dementsprechend in sehr effektiver Weise genutzt werden.

Der Behälter weist zumindest ein Fenster auf, welches durch das Wiederaufbereitungsgerät wahlweise geöffnet oder geschlossen werden kann. Hierzu kann der Behälter beispielsweise doppelwanding mit einer Außenhülle und einer Innenhülle ausgebildet sein, wobei sowohl die Außenhülle als auch die Innenhülle zumindest ein Fenster aufweisen und wobei die beiden Hüllen relativ zueinander bewegbar sind, um zum Öffnen bzw. Schließen des Behälters die Fenster fluchtend bzw. versetzt zueinander auszurichten. Dies kann in einfacher und eleganter Weise insbesondere dadurch erreicht werden, dass der Behälter zylinderförmig ausgebildet ist und die Außenhülle und Innenhülle gegeneinander verdrehbar sind.

Weiterhin ist vorzugsweise vorgesehen, dass beide Behälterteile manuell voneinander trennbar sind. Die an einem Behälterteil vorgesehen Kupplungselemente zum Anschließen der Instrumente sind dementsprechend sehr gut zugänglich, sodass beim Bestücken der Kupplungen mit den aufzubereitenden Instrumenten die Verletzungsgefahr reduziert wird. Im geschlossenen Zustand ist der Behälter vorzugsweise luftdicht geschlossen, sodass die wiederaufbereiteten Instrumente in sauberer und sicherer Weise zu Ihrem Einsatzort transportiert werden können.

Nachfolgend soll die Erfindung anhand der beiliegenden Zeichnung näher erläutert werden. Es zeigen:
- Figur 1: in Schnittdarstellung eine Prozess- bzw. Spülkammer eines erfindungsgemäßen Wiederaufbereitungsgeräts zum Desinfizieren, Sterilisieren und/oder Pflegen von zahnärztlichen Instrumenten; und
- Figur 2: ein Ausführungsbeispiel eines erfindungsgemäßen Aufnahmebehälters.

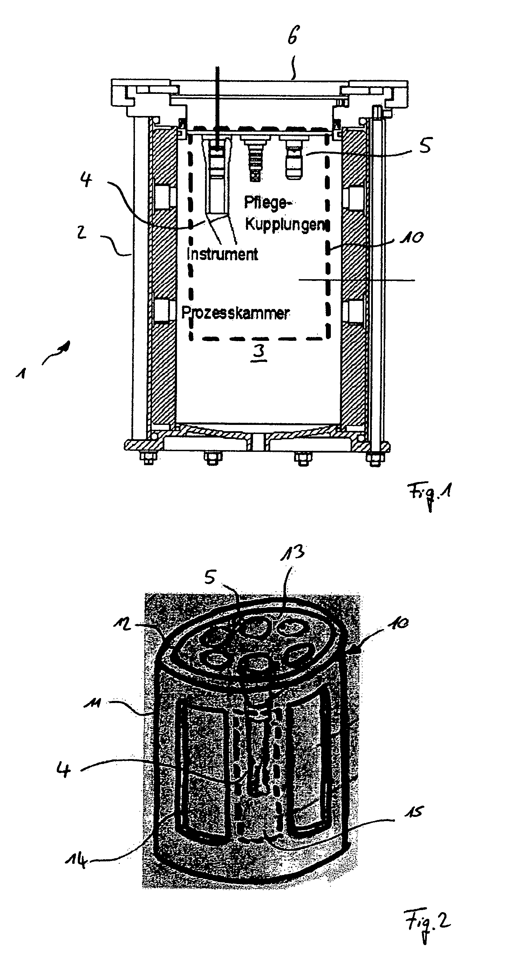

Figur 1 zeigt zunächst schematisch die Ausgestaltung eines Geräts zum Desinfizieren, Sterilisieren und/oder Pflegen von ärztlichen, insbesondere zahnärztlichen Instrumenten, wobei das Gerät nachfolgend allgemein mit dem Bezugszeichen 1 versehen ist. Zentrales Element des erfindungsgemäßen Pflegegeräts 1 ist ein Druckbehälter 2, der eine Prozess- bzw. Spülkammer 3 umschließt. In dieser Spülkammer 3 sind während des Prozessablaufs die zu reinigenden bzw. pflegenden Instrumente 4 angeordnet. Die Anordnung der Instrumente 4 erfolgt - wie nachfolgend noch näher beschrieben wird - hierbei mit Hilfe eines speziellen Aufnahmebehälters 10, der in Fig. 1 durch Strichlinien angedeutet ist und der mehrere Steckplätze bzw. Kupplungen 5 für die zu reinigenden Instrumente aufweist. Vorzugsweise sind unterschiedliche Kupplungen 5 vorgesehen, sodass Instrumente 4 mit Kupplungssystemen verschiedener Hersteller aufbereitet werden können. Der Aufnahmebehälter 10 stellt gemeinsam mit dem Deckel 6 des Geräts 1 die fluidische Ankopplung der zu reinigenden Instrumente 4 an ein Versorgungssystem sicher. Über in den Deckel 6 integrierte Verbindungsrohre können dann die einzelnen Instrumente 4 und deren Kanäle einzeln oder gemeinsam mit einem Reinigungs- und/oder Pflegemittel beaufschlagt werden.

Bevor der erfindungsgemäße Aufnahmebehälter 10 detaillierter besprochen wird, soll zunächst beispielshaft der Prozessablauf bei der Reinigung und/oder Pflege der Instrumente 4 beschrieben werden. Hierbei wird vor dem Start der Aufbereitung die Druckdichtigkeit der Prozesskammer 3 überprüft. Dabei wird sichergestellt, dass der Deckel 6 richtig eingesetzt und mit dem Druckbehälter 2 verriegelt ist. Auch eine korrekte Verbindung der Fluidleitungen zu den Kupplungen 5 hin wird überprüft.

Zur Wasserversorgung des Geräts 1 wird Leitungswasser vorzugsweise mittels einer Osmose-Anlage mit oder ohne nachgeschalteten Mischbett-Ionenaustauscher filtriert, wobei die gelösten Salze entfernt werden. Das Wasser bei einer Qualität von <15 µS/cm wird in einen geräteseitigen Vorratsbehälter geleitet, wobei der Füllstand über einen Niveauschalter, der als Schwimmerschalter ausgestaltet ist, und die Güte über einen Leitwertsensor kontrolliert wird. Der Einlass in den Vorratsbehälter ist aus hygienischen Gründen mit einer sogenannten freien Fallstrecke ausgestaltet.

Beim Aufbereiten der Instrumente werden dann nacheinander die folgenden Schritte ausgeführt:
a) Reinigen Zunächst wird Wasser aus dem zuvor beschriebenen Vorratsbehälter in die Prozesskammer 3 geleitet, wobei dies über eine Pumpe oder durch Ansaugen über Vakuum erfolgen kann. In der Prozesskammer 3 wird das Wasser mit Hilfe von Heizelementen auf etwa 45°C aufgeheizt. Dabei wird darauf geachtet, dass die Temperatur nicht oberhalb von 45°C liegt, um ein Koagulieren von Eiweiß zu verhindern. Das Wasser wird ferner mit Hilfe einer Pumpe umgewälzt und über Sprühdüsen, welche an der Mantelfläche des Druckbehälters 2 oder in einem Zentraldom angebracht sind, auf die Außenflächen der Instrumente 4 gerichtet, um diese zu reinigen. Dabei kann das Reinigungswasser durch die Instrumente 4 und/oder die Spraykanäle der Instrumente 4 und/oder zur Außenreinigung durch die Sprühdüsen der Prozesskammer 3 geleitet werden.
   Das Aufheizen des Waschmediums kann während der Umwälzung erfolgen, sodass die zu reinigenden Flächen zunächst mit kaltem Waschmedium gereinigt werden. Das Reinigungsmittel kann hierbei in Form von Pulver oder in Tablettenform in die Prozesskammer 3 zugegeben werden oder aus einem entsprechenden Vorratsbehälter zudosiert werden. Das Waschmedium kann dabei aus Tensiden bzw. Phosphaten bestehen und einen ph-Wert oberhalb von 10 aufweisen. Zur Beendigung des Waschvorgangs wird das Wasser aus dem Druckbehälter 2 abgelassen.
b) Klarspülen - Neutralisation
   In einem darauffolgenden Schritt wird dann das Wasser aus dem Vorratsbehälter in die Prozesskammer 3 geleitet und nun auf ca. 45°C bis 60°C aufgeheizt. Während des Umwälzenz des Wassers wird aus einem weiteren Vorratsbehälter Klarspüler bzw. Neutralisator zudosiert. Alternativ kann aufgrund der höheren Temperatur im Vergleich zu dem Schritt a) nunmehr auch eine zweite Komponente einer Reinigungstablette aufgelöst werden. Die Flüssigkeit wird wiederum parallel oder zeitversetzt bzw. im Intervallbetrieb durch die Instrumente 4 und die Spraykanäle geleitet bzw. über die Sprühdüsen auf die Außenflächen der Instrumente 4 gerichtet. Als Klarspüler bzw. Neutralisator kommt insbesondere Phosphorsäureester mit einem ph-Wert von 3 bis 5 zur Anwendung.
   Die Flüssigkeit kann wiederum aus dem Druckbehälter in die Kanalisation abgelassen werden oder verbleibt im Behälter, um beim späteren Pflegevorgang aus den Instrumenten 4 austretendes überschüssiges Pflegemittel aufzunehmen bzw. um die ölige Instrumentenaußenfläche mit warmer Flüssigkeit kurz abzuspülen. In diesem Fall wird die Flüssigkeit erst nach dem Pflegevorgang abgelassen, wobei es hilfreich sein kann, die Instrumente 4 mit Druckluft zu beaufschlagen, um ein Eindringen von Sprühwasser in das Innere der Instrumente 4 zu verhindern.
c) Pflegen
   In einem dritten Schritt wird aus einem Pflegemittelvorratsbehälter Pflegemittel in das Instrumenteninnere geleitet, sodass die Getriebe und Lagerstellen geschmiert werden. Das Pflegemittel kann dabei in flüssiger Form als Öl oder aus einer Druckdose in einen Druckluftstrahl injektiert werden. Es ist auch möglich, das Öl über das in der Druckdose enthaltene Treibmittel aufzuschäumen und das Instrumenteninnere mit diesem Öl-Luft-Schaum zu füllen. Die Luftbläschen kollabieren in diesem Fall verhältnismäßig rasch, sodass das Öl im gesamten Instrumenteninneren einen gleichförmigen dünnen Ölfilm bildet. Als Schmierstoffe kommen biologisch abbaubare Fettsäure-Esteröl/Weißöl-Gemische zur Anwendung.
d) Abspülen
   Nachdem zuvor beschriebenen Pflegevorgang können die Instrumente an der Außenfläche mit der noch im Behälter befindlichen Klarspülflüssigkeit abgespült werden. Alternativ hierzu wird über eine Pumpe frisches Wasser aus dem Vorratsbehälter der Prozesskammer 3 zugeführt und über die Sprühdüsen auf die Instrumentenaußenflächen gerichtet.
e) Sterilisation - Vorvakuum
   Zum Sterilisieren der Instrumente wird der Prozesskammer 3 frisches Wasser aus dem Vorratsbehälter zugeführt. In der Prozesskammer 3 ist zur Entlüftung eine Vakuumeinrichtung angeschlossen, wobei der Druck innerhalb der Prozesskammer 3 überwacht bzw. registriert wird.
   Mit Hilfe der Vakuumeinrichtung wird die Luft aus der Prozesskammer 3 abgesaugt. Das Vakuum wird durch Aufheizen des Wassers über Heizelemente bis auf Atmosphärendruck abgebaut. Die Prozesskammer 3 wird dann mit Wasserdampf befüllt, wobei sich dieser Vorgang je nach Sterilisationsprogramm mehrmals wiederholen kann.
   Das verdampfte Wasservolumen kann bei jedem Vakuumzyklus nachgefüllt werden, wobei alternativ hierzu auch die gesamte für die Dampferzeugung erforderliche Wassermenge gleich zu Beginn des Sterilisationszyklus in die Prozesskammer 3 eingebracht werden kann.
   Alternativ zur Dampferzeugung über in der Prozesskammer 3 befindliche Heizelemente kann Wasserdampf zum Druckausgleich bei der Entlüftung bzw. zur Sterilisation auch aus einem außerhalb der Prozesskammer 3 befindlichen Dampfdruckkessel zugeführt werden.
f) Trocknung und Kühlung
   Nach Abschluss der Sterilisation werden die Instrumente 4 getrocknet, in dem der in der Prozesskammer 3 befindliche Wasserdampf zur Kondensation gebracht wird. Dies wird dadurch erzielt, dass die Behälterwand oder in dem Behälter befindliche Elemente gekühlt werden, beispielsweise indem aus dem Vorratshälter entnommenes Wasser durch sie geleitet wird. Dabei kann das Wasser kontinuierlich oder intervallförmig zugeführt werden. Nach Beendigung des Kühlvorgangs wird das Wasser abgeleitet. Da nunmehr innerhalb der Kamer 3 eine Temperatur unterhalb von 50°C vorliegt, kann der Deckel 6 geöffnet werden. Der Aufbereitungszyklus für die Instrumente 4 ist hierdurch abgeschlossen.

Aus der obigen Schilderung ergibt sich, dass mit dem Gerät 1 eine vollautomatische Aufbereitung zahnärztlicher Instrumente ermöglicht wird. Eingriffe durch Bedienpersonal sind nicht erforderlich, sodass ein sehr komfortables System vorliegt. Dabei kann selbstverständlich auch von dem geschilderten Auflauf zum Aufbereiten der Instrumente abgewichen werden.

Die vorliegende Erfindung befasst sich insbesondere mit der Art und Weise der Bestückung der Kupplungen 5. Diese sind - wie bereits erwähnt - nicht unmittelbar an dem Pflegegerät 1 angeordnet, sondern stattdessen Bestandteil eines mit dem Gerät 1 lösbar verbindbaren Transportbehälters 10. Sinn und Zweck dieser Lösung ist, dass der Transportbehälter 10 von dem Gerät 1 entnommen werden und separat bestückt werden kann. Dies ist verhältnismäßig einfach durchzuführen, was zu einer Reduktion der Verletzungsgefahr, welche möglicherweise eine Infektion nach sich ziehen könnte, führt. Ein bevorzugtes Ausführungsbeispiel eines entsprechenden Behälters 10 ist in Figur 2 dargestellt und soll nachfolgend erläutert werden.

Der gleichzeitig als Halterung für die Instrumente in dem Gerät 1 dienende Behälter 10 besteht genau genommen aus zwei Behälterteilen 11 und 12, welche jeweils zylinderförmig ausgebildet sind und an einem ihrer beiden Enden geöffnet sind. Beide Behälterteile können wie in Figur 2 gezeigt derart zusammengesetzt werden, dass letztendlich ein geschlossener Hohlzylinder gebildet wird, wobei an einer Stirnwand 13 des inneren Behälterteils 12 die Kupplungen 5 für die Instrumente 4 angeordnet sind. Zum Bestücken der Kupplungen 5 können also einfach beide Behälterteile 11 und 12 voneinander getrennt werden, sodass die Kupplungen 5 gut erreichbar sind.

Eine weitere Besonderheit des erfindungsgemäßen Behälters 10 besteht ferner darin, dass dieser ein Öffnungssystem besitzt, welches es erlaubt, den Behälter 10 während der Wiederaufbereitung derart zu öffnen, dass die Instrumente 4 für die in die Spülkammer 3 eingebrachten Medien gut erreichbar sind. Dieses Öffnen und Schließen des Behälters 10 kann hierbei durch das Gerät 1 selbstständig vorgenommen werden.

Das zuvor angesprochene Öffhungssystem wird im dargestellten Ausführungsbeispiel dadurch realisiert, dass an den Umfangswänden der beiden Behälterteile 11 und 12 Fenster 14 bzw. 15 vorgesehen sind. Je nach Stellung der Behälterteile 11, 12 zueinander sind die Fenster 14, 15 fluchtend ausgerichtet oder versetzt zueinander angeordnet. Im ersten Fall werden dann durchgängige Fenster geschaffen, über welche eine Reinigung der Instrumente 4 ermöglicht ist. Im zweiten Fall ist die Umfangswand des Transportbehälters 10 vollständig geschlossen. Die Instrumente 4 sind hierbei von äußeren Einflüssen geschützt. Um den Behälter 10 von dem geschlossenen Transportzustand in den geöffneten Pflegezustand zu überführen, muss also lediglich durch das Wiederaufbereitungsgerät 1 eine Verdrehung beider Behälterteile 11 und 12 durchgeführt werden. Dies wird durch einen geeigneten Kopplungsmechanismus zwischen Behälter 10 und Gerät 1 erzielt.

Die Nutzung des erfindungsgemäßen Aufnahmebehälters 10 erfolgt also dadurch, dass der Benutzer den Behälter 10 öffnet und benutzte zahnärztliche Instrumente auf die Kupplungen 5 aufsteckt. Beide Behälterteile 11 und 12 werden nunmehr zusammengesetzt, wodurch der Behälter verschlossen wird. Ab diesem Zeitpunkt ist die Gefahr einer Verletzung und möglicherweise sogar einer Infektion gebannt. Der Behälter 10 wird dann in das Wiederaufbereitungsgerät 1 eingesetzt und das zuvor beschriebene Pflegeprogramm wird durchgeführt. Während dieser Zeit kann der Benutzter bereits einen weiteren Transportbehälter bestücken. Nach Ablauf des Reinigungs- bzw. Pflegeprogramms kann der Behälter 10 in einfacher Weise entnommen werden. Da er vorzugsweise im geschlossenen Zustand gegenüber der Umgebung abgedichtet ist, kann er auch zum längeren Lagern der gereinigten Instrumente genutzt werden.

Dementsprechend ermöglicht die erfindungsgemäße Lösung ein besonders einfaches Bestücken des Wiederaufbereitungsgeräts mit zu reinigenden bzw. zu pflegenden Instrumenten.

## Patentansprüche

1. Aufnahmebehälter (10) für ärztliche, insbesondere zahnärztliche Instrumente (4), wobei der Behälter (10) zur Verwendung in einem Wiederaufbereitungsgerät (1) vorgesehen ist, welches eine Kammer (3) zur Aufnahme des Behälters (10) sowie Mittel zum Desinfizieren, Sterilisieren und/oder Pflegen der Instrumente (4) aufweist, wobei der Behälter (10) derart gestaltet ist, dass er von einem Transportzustand, in dem der Behälter (10) geschlossen ist, in einen Pflegezustand, in dem der Behälter (10) derart geöffnet ist, dass darin angeordnete Instrumente (4) zugänglich sind, überführbar ist, und
wobei der Behälter (10) derart mit dem Wiederaufbereitungsgerät (1) koppelbar ist, dass dieses in der Lage ist, den in die Kammer eingesetzten Behälter (10) wahlweise von dem Transportzustand in den Pflegezustand bzw. umgekehrt zu überführen, **dadurch gekennzeichnet,**
**dass** der Behälter (10) zumindest ein Fenster (14, 15) aufweist, welches durch das Wiederaufbereitungsgerät (1) wahlweise geöffnet oder geschlossen werden kann.

2. Aufnahmebehälter nach Anspruch 1,
**dadurch gekennzeichnet,**
**dass** der Behälter (10) doppelwandig mit einer Außenhülle (11) und einer Innenhülle (12) ausgebildet ist, wobei sowohl die Außenhülle (11) als auch die Innenhülle (12) zumindest ein Fenster aufweisen,
wobei die beiden Hüllen (11, 12) relativ zueinander bewegbar sind, um zum Öffnen bzw. Schließen des Behälters (10) die Fenster fluchtend bzw. versetzt zueinander auszurichten.

3. Aufnahmebehälter nach Anspruch 2,
**dadurch gekennzeichnet,**
**dass** der Behälter (10) zylinderförmig ausgebildet ist und die Außenhülle (11) und Innenhülle (12) gegeneinander verdrehbar sind.

4. Aufnahmebehälter nach Anspruch 3,
**dadurch gekennzeichnet,**
**dass** die Außenhülle (11) und die Innenhülle (12) manuell voneinander trennbar sind.

5. Aufnahmebehälter nach einem der vorherigen Ansprüche,
**dadurch gekennzeichnet,**
**dass** der Behälter (10) im geschlossenen Zustand luftdicht geschlossen ist.

6. Aufnahmebehälter nach einem der vorherigen Ansprüche,
**dadurch gekennzeichnet,**
**dass** der Behälter (10) Kupplungselemente (5) zum Anschließen von Instrumenten (4) aufweist, wobei die Kupplungselemente (5) mit Medienleitungen des Wiederaufbereitungsgeräts koppelbar sind.

7. System zum Desinfizieren, Sterilisieren und/oder Pflegen von ärztlichen, insbesondere zahnärztlichen Instrumenten mit
einem Wiederaufbereitungsgerät (1), welches eine Kammer (3) zur Aufnahme der Instrumente (4) sowie Mittel zum Desinfizieren, Sterilisieren und/oder Pflegen von in der Kammer (3) befindlichen Instrumenten (4) aufweist,
sowie einer von dem Wiederaufbereitungsgerät (1) trennbaren Halterung zur Anordnung der Instrumente (4) in der Kammer (3) des Wiederaufbereitungsgeräts (1), **dadurch gekennzeichnet,**
**dass** die Halterung als Behälter (10) gemäß einem der vorherigen Ansprüche ausgebildet ist.

## Claims

1. A receiving container (10) for medical, in particular dental, instruments (4), wherein the container (10) is provided for use in a reconditioning device (1) which has a chamber (3) to receive the container (10) and also means for disinfecting, sterilizing and/or maintaining the instruments (4), wherein the container (10) is configured in such a way that it can be transferred from a transportation state, in which the container (10) is closed, into a maintenance state, in which the container (10) is opened in such a way that instruments (4) that are arranged therein are accessible, and
wherein the container (10) can be coupled with the reconditioning device (1) in such a way that the latter is able to transfer the container (10), which has been inserted into the chamber, selectively from the transportation state into the maintenance state or *vice versa,*
**characterised in that**
the container (10) has at least one window (14, 15) which can be selectively opened or closed by means of the reconditioning device (1).

2. A receiving container according to claim 1,
**characterised in that**
the container (10) is formed with two walls with an outer sleeve (11) and an inner sleeve (12), wherein both the outer sleeve (11) and the inner sleeve (12) have at least one window,
wherein the two sleeves (11, 12) can be moved relative to each other in order to adjust the windows so that they are in alignment or offset with respect to each other in order to open or close the container (10).

3. A receiving container according to claim 2,
**characterised in that**
the container (10) is formed cylindrically, and the outer sleeve (11) and inner sleeve (12) can be twisted in relation to each other.

4. A receiving container according to claim 3,
**characterised in that**
the outer sleeve (11) and the inner sleeve (12) can be separated from each other manually.

5. A receiving container according to one of the previous claims, **characterised in that**
the container (10) is closed in an air-tight manner in the closed state.

6. A receiving container according to one of the previous claims,
**characterised in that**
the container (10) has coupling elements (5) for the connection of instruments (4), wherein the coupling elements (5) can be coupled with media lines of the reconditioning device.

7. A system for disinfecting, sterilizing and/or maintaining medical, in particular dental, instruments having
a reconditioning device (1) which has a chamber (3) to receive the instruments (4) and also means to disinfect, sterilize and/or maintain instruments (4) that are located in the chamber (3),
and also a holding support, which can be separated from the reconditioning device (1), for the arrangement of the instruments (4) in the chamber (3) of the reconditioning device (1),
**characterised in that**
the holding support is formed as a container (10) in accordance with one of the previous claims.

## Revendications

1. Contenant formant un réceptacle (10) pour des instruments (4) médicaux, en particulier dentaires, dans lequel le contenant (10) se destine à être utilisé dans un appareil de retraitement (1), qui présente un compartiment (3) servant à recevoir le contenant (10) ainsi que des moyens servant à désinfecter, stériliser et/ou entretenir les instruments (4), dans lequel le contenant (10) est configuré de telle manière qu'il peut être amené d'un état de transport, dans lequel le contenant (10) est fermé, dans un état d'entretien, dans lequel le contenant (10) est ouvert de telle manière que les instruments (4) disposés dans ledit contenant sont accessibles, et
dans lequel le contenant (10) peut être couplé à l'appareil de retraitement (1) de telle manière qu'il est en mesure de transférer le contenant (10) inséré dans le compartiment au choix de l'état de transport dans l'état d'entretien ou inversement,
**caractérisé en ce**
**que** le contenant (10) présente au moins une fenêtre (14, 15), qui peut être au choix ouverte ou fermée par l'appareil de retraitement (1).

2. Contenant formant un réceptacle selon la revendication 1,
**caractérisé en ce**
**que** le contenant (10) est réalisé de manière à présenter une paroi double pourvue d'une enveloppe extérieure (11) et d'une enveloppe intérieure (12), dans lequel aussi bien l'enveloppe extérieure (11) que l'enveloppe intérieure (12) présentent au moins une fenêtre,
dans lequel les deux enveloppes (11, 12) peuvent être déplacées l'une par rapport à l'autre pour orienter la fenêtre de manière alignée ou de manière décalée afin d'ouvrir ou de fermer le contenant (10).

3. Contenant formant un réceptacle selon la revendication 2,
**caractérisé en ce**
**que** le contenant (10) est réalisé de manière à présenter une forme cylindrique, et en ce que l'enveloppe extérieure (11) et l'enveloppe intérieure (12) peuvent être tournées l'une par rapport à l'autre.

4. Contenant formant un réceptacle selon la revendication 3, caractérisé en
que l'enveloppe extérieure (11) et l'enveloppe intérieure (12) peuvent être séparées l'une de l'autre manuellement.

5. Contenant formant un réceptacle selon l'une quelconque des revendications précédentes,
**caractérisé en ce**
**que** le contenant (10) est fermé de manière étanche à l'air dans l'état fermé.

6. Contenant formant un réceptacle selon l'une quelconque des revendications précédentes,
**caractérisé en ce**
**que** le contenant (10) présente des éléments de couplage (5) servant à raccorder des instruments (4), dans lequel les éléments de couplage (5) peuvent être couplés à des conduites de fluide de l'appareil de retraitement.

7. Système servant à désinfecter, stériliser et/ou entretenir des instruments médicaux, en particulier dentaires, comprenant
un appareil de retraitement (1), qui présente un compartiment (3) servant à recevoir les instruments (4) ainsi que des moyens servant à désinfecter, stériliser et/ou entretenir des instruments (4) se trouvant dans le compartiment (3),
ainsi qu'un support pouvant être séparé de l'appareil de retraitement (1), servant à disposer les instruments (4) dans le compartiment (3) de l'appareil de retraitement (1),
**caractérisé en ce**
**que** le support est réalisé sous la forme d'un contenant (10) selon l'une quelconque des revendications précédentes.
